(19) Europäisches Patentamt
European Patent Office
Office européen des brevets

(11) Veröffentlichungsnummer: **0 037 982**
**A1**

# (12) EUROPÄISCHE PATENTANMELDUNG

(21) Anmeldenummer: **81102514.7**

(22) Anmeldetag: **03.04.81**

(51) Int. Cl.³: **B 01 J 29/28**, C 01 B 33/28

(30) Priorität: **16.04.80 DE 3014637**

(43) Veröffentlichungstag der Anmeldung: **21.10.81**
**Patentblatt 81/42**

(84) Benannte Vertragsstaaten: **BE DE FR GB IT NL**

(71) Anmelder: **BAYER AG, Zentralbereich Patente, Marken und Lizenzen, D-5090 Leverkusen 1, Bayerwerk (DE)**

(72) Erfinder: **Weisser, Jürgen, Dr., Südstrasse 15, D-4047 Dormagen 11 (DE)**
Erfinder: **Grolig, Johann, Dr., Heinrich-Lübke-Strasse 22, D-5090 Leverkusen 1 (DE)**
Erfinder: **Scharfe, Gerhard, Dr., Berta-von-Suttner-Strasse 69, D-5090 Leverkusen 1 (DE)**

(54) **Verfahren zur Herstellung geformter Katalysatoren auf der Basis kristalliner Aluminiumsilikate und deren Verwendung.**

(57) Die vorliegende Erfindung betrifft ein Verfahren zur Herstellung von geformten Katalysatoren mit einem minimalen Durchmesser über 1 mm und einem maximalen Durchmesser von unter 20 mm auf der Basis kristalliner Aluminiumsilikate, bei dem man Formkörper mit einem minimalen Durchmesser von über 1 mm und einem maximalen Durchmesser von unter 20 mm, die im wesentlichen aus amorphem Aluminiumsilikat mit einem Aluminiumoxidgehalt von 0,1 bis 20 Gew.-% bestehen, zusammen mit aliphatischen $C_2$- bis $C_{24}$-Aminen oder cycloaliphatischen $C_4$- bis $C_{24}$-Aminen und/oder Ammoniumsalzen entsprechend diesen aliphatischen und/oder cycloaliphatischen Aminen und/oder den aus diesen Aminen durch Quarternierung erhältlichen quarternären Ammoniumverbindungen, in einem wäßrigen Milieu, bei einem pH-Wert von größer als 8, Temperaturen von 50 bis 250 °C aussetzt und anschließend auswäscht, trocknet und bei 400 bis 600 °C calciniert. Gegenüber bekannten Verfahren zur Herstellung derartiger Katalysatoren, bei denen von löslichen oder teilweise löslichen Kieselsäuren oder Kieselsäurederivaten ausgegangen wird, haben die erfindungsgemäß hergestellten Katalysatoren den Vorteil einer einfachen Zugänglichkeit und eine Reihe weiterer Vorteile.

EP 0 037 982 A1

BAYER AKTIENGESELLSCHAFT 5090 Leverkusen, Bayerwerk
Zentralbereich
Patente, Marken und Lizenzen Gai/kl-c

# Verfahren zur Herstellung geformter Katalysatoren auf der Basis kristalliner Aluminiumsilikate und deren Verwendung

Die vorliegende Erfindung betrifft ein Verfahren zur Herstellung geformter Katalysatoren auf der Basis kristalliner Aluminiumsilikate aus amorphen Aluminiumsilikaten mit einem Aluminiumoxidgehalt von 0,1 bis 20 Gew.-%, sowie deren Verwendung als Festbettkatalysatoren.

Es ist bekannt, daß man zur Herstellung kristalliner Aluminiumsilikate, die eine katalytische Aktivität aufweisen, lösliche oder zumindest teilweise lösliche Formen von Kieselsäure einsetzen kann. Geeignet sind beispielsweise Natriumsilikat, Kieselsol oder reaktives Siliciumdioxidhydrat (siehe beispielsweise US-PS 3 702 886, US-PS 3 709 979, US-PS 4 025 571, US-PS 4 025 572, DE-OS 2 548 695 und DE-OS 2 548 697).

Als weitere Reaktionskomponenten sind lösliche Aluminiumverbindungen, z.B. Aluminate oder mineralsaure Aluminiumsalze erforderlich, die in Form einer wäßrigen Lösung und zusammen mit einem weiteren Reagenz eingesetzt werden. Bei letzterem handelt es sich in der Regel um eine organische stickstoff- oder phosphorhaltige Verbindung (siehe beispielsweise US-PS 3 702 886, US-PS 3 709 979, US-PS 4 108 881, DE-OS 2 748 278 und die Europäische Patentanmeldung mit der Veröffentlichungsnummer 0002899).

Diese Verfahren haben den entscheidenden Nachteil, daß das resultierende kristalline Aluminiumsilikat in Form einer wäßrigen Suspension anfällt, die häufig schwer zu filtrieren ist. Ebenso läßt sich der Filterkuchen sehr oft nur unter erheblichen Schwierigkeiten neutral waschen. Weiterhin ist nachteilig, daß das Reaktionsprodukt nach dem Filtrieren, Auswaschen und Calcinieren stets als mikrokristallines Pulver mit einer Partikelgröße von unter 20 µm, häufig sogar unter 10 µm erhalten wird.

In dieser Form können die kristallinen Aluminiumsilikate nicht als Festbettkatalysatoren verwendet werden. Um sie technisch einsetzen zu können, muß in nachfolgenden Arbeitsoperationen eine mechanische Formgebung erfolgen. Das kann durch Verpressen, Verpillen, Extrudieren oder durch Suspendieren, Agglomerisieren und Sprühtrocknen erfolgen. Zum Erreichen einer für die technische Anwendung notwendigen Festigkeit sind für diese Operationen Zusätze erforderlich, beispielsweise Schmier- oder Verfestigungsmittel, Binder und/oder Inertmaterialien. Diese Zusätze können jedoch einen nachteiligen Einfluß auf die Eigenschaften des erwünschten Katalysators ausüben und setzen in jedem Falle den Anteil an katalytisch aktiven Bestandteilen des Produkts herab.

Werden anstelle von löslichen oder teilweise löslichen Formen von Kieselsäure natürlich vorkommende Bodenmineralien, z.B. Smectit, als Rohstoffe für die Herstellung kristalliner und katalytisch aktiver Aluminiumsilikate verwendet, so resultieren Reaktionsprodukte, die nur zum Teil aus dem angestrebten kristallinen Silikat bestehen. So zeigt beispielsweise die US-PS 3 976 598, daß trotz Einstellung optimaler Reaktionsbedingungen hinsichtlich der Bildung eines aktiven Katalysatormaterials das Reaktionsprodukt unbefriedigend ist:

Ein für die Umwandlung von Methanol in Kohlenwasserstoffe geeignetes kristallines Aluminiumsilikat vom Typ Zeolith ZSM-5 ist nach diesem Verfahren nur in einer Konzentration von ca. 30 % im Reaktionsprodukt enthalten. Der Rest besteht hauptsächlich aus Zeolith P und ist somit für die angestrebte katalytische Verwendung nicht geeignet.

Soll aus diesem heterogenen Reaktionsprodukt ein Festbettkatalysator für die Umwandlung von Methanol in Kohlenwasserstoffe hergestellt werden, so reduziert sich der Anteil des aktiven Katalysators noch weiter, da für eine mechanische Formgebung in der Regel zumindest Bindemittel zugesetzt werden müssen.

Ein anderer, ebenfalls nachteiliger Weg zu geformten Katalysatoren ist die Verformung von Bodenmineralien mit einem hohen Aluminiumoxidgehalt, wie z.B. Ton oder Kaolin und die anschließende Umwandlung der Formkörper in ein als Katalysator, z.B. für die Methanolumwandlung, geeignetes kristallines Aluminiumsilikat.

Abgesehen von den mineralischen Verunreinigungen, die in Bodenmaterialien fast immer enthalten sind und die sich bei einer katalytischen Verwendung störend auswirken können, müssen notwendigerweise erhebliche Mengen von löslichen bzw. hochreaktiven Kieselsäurekomponenten (z.B. Wasserglas, Kieselsol, Aerosil ®) dem Ausgangsmaterial zugeführt werden, damit das Verhältnis von $SiO_2:Al_2O_3$ in einem für die Bildung des erwünschten Katalysatormaterials günstigen Bereich liegt.

Hinzu kommt, daß bei dieser Verfahrensweise das Verhältnis der Reaktionskomponenten zueinander und sogar die verwendete Wassermenge äußerst kritisch ist. Schon relativ geringe Änderungen können hier bewirken, daß anstelle des erwünschten kristallinen Aluminiumsilikats vom Typ ZSM-5 ausschließlich eine Mischung der für katalytische Zwecke wenig geeigneten Zeolithe-P und -S entsteht.

Es wurde nun ein Verfahren zur Herstellung von geformten Katalysatoren mit einem minimalen Durchmesser über 1 mm und einem maximalen Durchmesser von unter 20 mm auf der Basis kristalliner Aluminiumsilikate gefunden, das dadurch gekennzeichnet ist, daß man Formkörper mit einem minimalen Durchmesser von über 1 mm und einem maximalen Durchmesser von unter 20 mm, die im wesentlichen aus amorphem Aluminiumsilikat mit einem Aluminiumoxidgehalt von 0,1 bis 20 Gew.-% bestehen, zusammen mit aliphatischen $C_2$- bis $C_{24}$-Aminen oder cycloaliphatischen $C_4$- bis $C_{24}$-Aminen und/oder Ammoniumsalzen entsprechend diesen aliphatischen und/oder cycloaliphatischen Aminen und/oder den aus diesen Aminen durch Quarternierung erhältlichen quarternären Ammoniumverbindungen, in einem wäßrigen Milieu, bei einem pH-Wert von größer als 8, Temperaturen von 50 bis 250°C aussetzt und anschließend auswäscht, trocknet und bei 400 bis 600°C calciniert.

Die erfindungsgemäß einzusetzenden Formkörper, die im wesentlichen aus amorphem Aluminiumsilikat mit einem Aluminiumoxidgehalt von 0,1 bis 20 Gew.-% bestehen, können eine beliebige Gestalt innerhalb der angegebenen Abmessungen aufweisen. Insbesondere sind diejenigen geometrischen Formen geeignet, die für Festbettkatalysatoren in Frage kommen, beispielsweise Kugeln, Tabletten, Strangpreßlinge, zylindrische Extrudate, Würstchen, Tropfen, Röhrchen oder Waben. Vorzugsweise werden kugelförmige oder annähernd kugelförmige Formkörper eingesetzt, die einen Durchmesser im Bereich von 2 bis 15 mm aufweisen.
Beispielsweise lassen sich für das erfindungsgemäße Verfahren Formkörper aus amorphen Aluminiumsilikaten verwenden, die auf Basis sogenannter Dreischichtmineralien (z.B. Bentonit oder Montmorillonit) hergestellt werden und in denen durch saures Herauslösen der vorwiegend aus Eisen- und Aluminiumoxid bestehenden Octaederschicht das Kristallgitter weitgehend amorph geworden ist.

Erfindungsgemäß werden Formkörper eingesetzt, die im wesentlichen aus amorphem Aluminiumsilikat bestehen, das einen Aluminiumoxidgehalt im Bereich von 0,1 bis 20 Gew.-% aufweist. Vorzugsweise beträgt der Aluminiumoxidgehalt 1 bis 15 Gew.-%. Besonders bevorzugt sind Aluminiumoxidgehalte von 1 bis 6 Gew.-%.
Zum Einsatz in das erfindungsgemäße Verfahren geeignete Formkörper sind im Handel erhältlich.
Es kann vorteilhaft sein, die Formkörper vor der erfindungsgemäßen Behandlung mit Aminen, Ammoniumsalzen und/oder quarternären Ammoniumverbindungen in einen Zustand erhöhter Reaktionsbereitschaft zu bringen. Dies kann beispielsweise erfolgen, indem man die Formkörper

glüht und gegebenenfalls anschließend entgast. Das Glühen kann beispielsweise bei Temperaturen im Bereich 600 bis 1100°C für beispielsweise 1 bis 6 Stunden erfolgen. Die Entgasung kann beispielsweise erfolgen, indem man die Formkörper nach dem Glühen in ein evakuierbares Gefäß einbringt und dieses evakuiert.

Die erfindungsgemäße Behandlung mit Aminen, Ammoniumsalzen und/oder quartären Ammoniumverbindungen wird vorzugsweise mit einer oder mehreren Verbindungen aus einer oder mehreren der folgenden Gruppen vorgenommen:

Monoamine der Formel

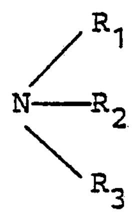

(I)

in der

$R_1$, $R_2$ und $R_3$ gleich oder verschieden sein können und für $C_2$-$C_6$-Alkyl, $C_2$-$C_6$-Hydroxyalkyl oder $C_2$-$C_6$-Aminoalkyl stehen und in der $R_1$ und/oder $R_2$ auch für Wasserstoff steht.

Diamine der Formel

$$(R_4)_2N-[C(R_5)_2]_n-N(R_6)_2 \qquad (II)$$

in der

$R_4$, $R_5$ und $R_6$ gleich oder verschieden sein können und für $C_1$-$C_2$-Alkyl, $C_1$-$C_2$-Hydroxyalkyl, $C_1$-$C_2$-Aminoalkyl oder Wasserstoff stehen und in der n für eine ganze Zahl von 2 bis 6 steht.

Monoammoniumsalze der Formel

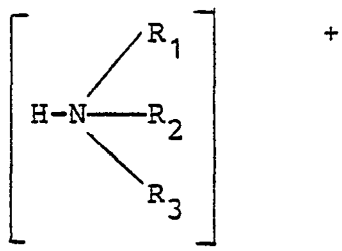

$X^-$ (III)

in der

$R_1$, $R_2$ und $R_3$ die bei Formel (I) angegebene Bedeutung haben und in der X für Cl, Br, J, $HSO_4$, $HCO_3$, $H_2PO_4$ oder $BF_4$ steht.

Monoammoniumsalze der Formel

$$[H(R_4)_2N-[C(R_5)_2]_n-N(R_6)_2]^+ \quad X^- \qquad (IV)$$

in der

$R_4$, $R_5$, $R_6$ und n die bei Formel (II) angegebene Bedeutung haben und in der X die bei Formel (III) angegebene Bedeutung hat.

Quarternäre Ammoniumverbindungen der Formel

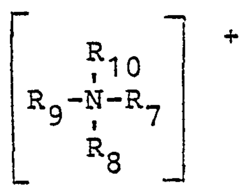

$X^-$ (V)

in der

$R_7$, $R_8$, $R_9$ und $R_{10}$ gleich oder verschieden sein können und für $C_2$-$C_4$-Alkyl, $C_2$-$C_4$-Hydroxyalkyl oder $C_2$-$C_4$-Aminoalkyl stehen,

oder in der

zwei der Reste $R_7$, $R_8$, $R_9$ und $R_{10}$ für $C_1$-$C_4$-Alkyl, $C_2$-$C_4$-Hydroxyalkyl oder $C_2$-$C_4$-Aminoalkyl und die beiden anderen Reste für $C_2$-$C_{12}$-Alkyl, Benzyl, $C_2$-$C_{12}$-Hydroxyalkyl, $C_2$-$C_{12}$-Aminoalkyl oder $C_4$-$C_8$-Cycloalkyl stehen, und

X OH ist oder die bei Formel (III) angegebene Bedeutung hat,

wobei quarternäre Ammoniumverbindungen mit mehr als insgesamt 24 C-Atomen ausgeschlossen sind.

Quarternäre Ammoniumverbindungen der Formel

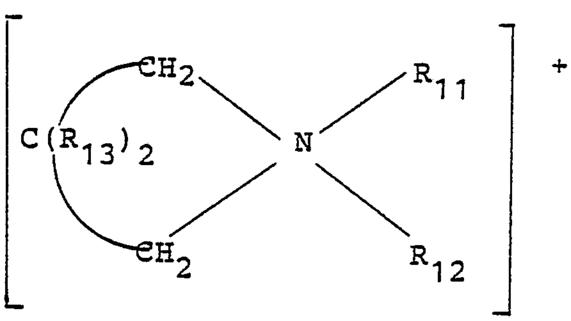

$X^-$ (VI)

in der

$R_{11}$ und $R_{12}$ gleich oder verschieden sein können und für $C_1$-$C_8$-Alkyl, $C_2$-$C_8$-Hydroxyalkyl oder

$C_2$-$C_8$-Aminoalkyl stehen,

$R_{13}$ für Wasserstoff oder $C_1$-$C_8$-Alkyl steht,

und X OH ist oder die bei Formel (III) angegebene Bedeutung hat,

wobei quarternäre Ammoniumverbindungen mit mehr als insgesamt 24 C-Atomen ausgeschlossen sind.

Quarternäre Ammoniumverbindungen der Formel

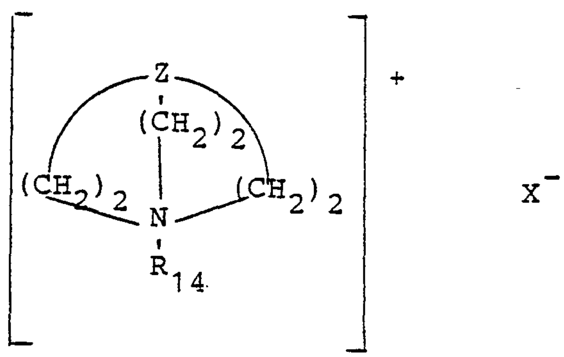

(VII)

in der

$R_{14}$ für $C_1$-$C_8$-Alkyl oder 2-Hydroxyethyl steht,

Z für Stickstoff, $-CR_{15}$ oder $-NR_{16}^{+}\ X^{-}$ steht, wobei $R_{15}$ für $C_1$-$C_8$-Alkyl steht und $R_{16}$ die gleiche Bedeutung hat, wie vorstehend für $R_{14}$ angegeben und

X OH ist oder die bei Formel (III) angegebene Bedeutung hat,

wobei quarternäre Ammoniumverbindungen mit insgesamt mehr als 24 C-Atomen ausgeschlossen sind.

Innerhalb der Verbindungen gemäß den Formeln (I) bis (VII) sind solche bevorzugt, die insgesamt 2 bis 16 C-Atome enthalten.

Besonders bevorzugt werden als Amine, Ammoniumsalze und/oder quarternäre Ammoniumverbindungen eine oder mehrere der folgenden Verbindungen eingesetzt: Tetrapropylammoniumhydroxid, -chlorid, -bromid, -jodid, Tetrabutylammoniumhydroxid, -chlorid, -bromid, -jodid, Tris-(2-hydroxyethyl)-n-propylammoniumhydroxid, -chlorid, -bromid, -jodid, sowie Tetramethylendiamin, Pentamethylendiamin, Hexamethylendiamin, n-Propylamin, n-Butylamin, Ethanolamin, Diethanolamin, Triethanolamin, wobei die genannten Amine in freier Form oder an einem Stickstoffatom protoniert und mit Chlorid, Bromid, Jodid, Hydrogensulfat, Dihydrogenphosphat, Hydrogencarbonat und/oder Tetrafluoroborat als Anion eingesetzt werden können.

Ganz besonders bevorzugt werden Tetrapropylammoniumbromid, Tetrabutylammoniumbromid und Tris-(2-hydroxyethyl)-n-propylammoniumhydroxid eingesetzt.

Die Menge, in der erfindungsgemäß Amine, Ammoniumsalze und/oder quarternäre Ammoniumverbindungen (im folgenden gemeinsam als Stickstoffverbindungen bezeichnet) eingesetzt werden können, kann in einem weiten Bereich variiert werden. Beispielsweise kann man die Stickstoffverbindungen in einer Menge von 5 bis 100 Gew.-%, bezogen auf die eingesetzten Formkörper, einsetzen. Vorzugsweise setzt man 10 bis 50 Gew.-% einer oder mehrerer Verbindungen aus der Gruppe der zuvor beschriebenen Stickstoffverbindungen ein, bezogen auf eingesetzte Formkörper.

Die Menge Wasser, die erfindungsgemäß einzusetzen ist, kann ebenfalls in einem weiten Bereich variiert werden. Es ist vorteilhaft, mindestens soviel Wasser einzusetzen, daß das Gemisch aus Wasser und Stickstoffverbindung(en) ausreicht, um die Poren der eingesetzten Formkörper vollständig zu füllen. Selbstverständlich können auch größere Mengen an Wasser eingesetzt werden, beispielsweise so viel, daß die eingesetzten Formkörper völlig bedeckt werden oder in einer Menge, die einem Vielfachen des Porenvolumens der eingesetzten Formkörper entspricht.

Für das erfindungsgemäße Verfahren ist es wesentlich, daß das Gemisch aus Stickstoffverbindung(en) und Wasser einen pH-Wert von größer als 8 aufweist. Vorzugsweise beträgt der pH-Wert dieses Gemisches mindestens 9, besonders bevorzugt mindestens 10.

Zur Erreichung von höheren pH-Werten kann es erforderlich sein, dem Gemisch aus Stickstoffverbindung(en) und Wasser alkalisch reagierende Stoffe zuzusetzen. Außer stark alkalisch reagierenden Verbindungen aus der Gruppe der zuvor beschriebenen Stickstoffverbindungen kommen hierfür auch die verschiedensten organischen und/oder anorganischen Verbindungen mit ausreichender Alkalität

in Frage. Beispiele für derartige organische und anorganische Verbindungen sind Amine, wie Methyl-, Dimethyl-, Trimethyl-, Ethyl-, Diethyl-, Triethyl-, Methylethyl-, Propyl- und Butylamin, Anilin, Methyl-, Ethyl-, Dimethyl-, Diethyl-, Methylethylanilin, Chinolin und Pyridin; anorganische Oxide und Hydroxide, wie Alkali- und Erdalkalioxide und Alkali- und Erdalkalihydroxide, insbesondere Natrium-, Kalium-, Lithium-, Magnesium-, Calcium- und Bariumoxid oder -hydroxid; Salze aus starken Basen und schwachen Säuren, wie Carbonate, Phosphate, Silikate, Acetate, Fluoride, Stannite, Stannate, Borate, Wolframate, Molybdate, Sulfide, Sulfite von Natrium, Kalium, Lithium, Magnesium, Calcium und Barium.
Vorzugsweise kommen als Zusätze solche in Frage, die in Wasser gut löslich sind und mit den Stickstoffverbindungen und den eingesetzten Formkörpern nicht in unerwünschter Weise reagieren.

Auch aus anderen Gründen kann es vorteilhaft sein, dem Gemisch aus Stickstoffverbindung(en) und Wasser Zusätze zuzufügen. So wurde beispielsweise festgestellt, daß bei Zusätzen von Alkalicarbonaten, Alkaliphosphaten, Alkalifluoriden, Alkalisulfiden, Alkalicarboxylaten, Alkalisilikaten und/oder Alkaliwolframaten geformte Katalysatoren auf der Basis kristalliner Aluminiumsilikate erhalten werden können, die eine besonders gute Bruchhärte aufweisen. Dies ist insbesondere bei Zusätzen von Alkaliphosphaten, Alkalicarbonaten, Alkalisilikaten, Alkalisulfiden und/oder Alkaliwolframaten der Fall.

Die Zusätze zur Erhöhung des pH-Wertes und/oder zur Erzielung guter Bruchhärten können in weiten Grenzen variiert werden. Im allgemeinen sind Zusätze in einer Menge ausreichend, die bezogen auf die eingesetzten Formkörper 0,01 bis 10, vorzugsweise 0,1 bis 5 Gew.-% Hydroxidionen freisetzen können.

Die Stickstoffverbindung(en), das Wasser und die einzusetzenden Formkörper, sowie die gegebenenfalls einzusetzenden Zusätze können in beliebiger Reihenfolge zusammengegeben werden. Im allgemeinen geht man so vor, daß man aus Wasser, Stickstoffverbindung(en) und gegebenenfalls einem oder mehreren Zusätzen eine Lösung oder Suspension herstellt und die einzusetzenden Formkörper damit übergießt.

Nach dem Zusammengeben von Wasser, Stickstoffverbindung(en), einzusetzenden Formkörpern und gegebenenfalls Zusätzen wird das dabei gebildete Reaktionsgemisch Temperaturen im Bereich 50 bis 250°C, vorzugsweise im Bereich 80 bis 180°C, ausgesetzt. Dabei wird das amorphe Ausgangsmaterial unter Beibehaltung seiner äußeren Form zum größten Teil oder vollständig in kristallines Aluminiumsilikat überführt. Die Umwandlungsgeschwindigkeit hängt im wesentlichen von der Beschaffenheit des Ausgangsmaterials und der Temperatur ab. Man kann z.B. durch Debye-Scherrer (Röntgenbeugungs-)Analyse von Proben den jeweils erreichten Kristallinitätsgrad feststellen. Mit steigender Reaktionstemperatur nimmt die notwendige Behandlungszeit stark ab. Im allgemeinen können gute Ergebnisse erhalten werden, wenn man das Reaktionsgemisch beispielsweise 20 bis 60 Stunden bei ca. 180°C oder etwa 80 bis 150 Stunden bei ca. 120°C oder etwa 350 bis 500 Stunden bei ca. 80°C beläßt. In einzelnen Fällen können jedoch auch von diesen Richtwerten mehr oder weniger stark abweichende Behandlungszeiten notwendig sein.

Wenn die Behandlung bei Temperaturen vorgenommen werden soll, bei denen Teile des Reaktionsgemisches unter Normaldruck flüchtig sind, so wird zweckmäßigerweise in einem druckbeständigen, verschließbaren Gefäß gearbeitet. Es ist nicht notwendig, während der Behandlung zu rühren.

Wenn durch die Behandlung der gewünschte Kristallinitätsgrad erreicht worden ist, so wird das feste Reaktionsprodukt zunächst von der gegebenenfalls vorhandenen überschüssigen wäßrigen Phase abgetrennt, beispielsweise durch eine mechanische Abtrennung wie Dekantieren oder Filtrieren. Die abgetrennte wäßrige Phase kann, gegebenenfalls nach Ergänzung der verbrauchten Bestandteile, wiederverwendet werden. Es ist dann, auch wenn nach der Behandlung keine mechanisch abtrennbare wäßrige Phase vorhanden war, noch erforderlich, das feste Reaktionsprodukt bei 400 bis 600°C zu calcinieren. Diese Calcinierung wird durchgeführt, nachdem das feste Reaktionsprodukt mit Wasser neutral gewaschen und getrocknet worden ist.

Man erhält so geformtes, zum größten Teil oder vollständig kristallines Aluminiumsilikat, das meist schon eine gewisse katalytische Aktivität aufweist. In vielen Fällen kann die katalytische Aktivität noch verbessert werden.

Es ist deshalb vorteilhaft, das so erhaltene geformte, zum größten Teil oder vollständig kristalline Aluminiumsilikat in an sich bekannter Weise einem Ionenaustausch mit Ammoniumionen oder Wasserstoffionen zu unterziehen und es anschließend nochmals zu calcinieren.

So behandelte Produkte weisen im allgemeinen eine gute katalytische Aktivität auf. Sie eignen sich insbesondere als Katalysatoren für die Umwandlung von Methanol und/oder Dimethylether in Kohlenwasserstoffe, als Crackkontakte, für die Alkylierung, Disproportionierung und/oder Isomerisierung von Aromaten und für die Aromatisierung von Olefinen. Vorzugsweise werden derartige Produkte für die Umwandlung von Methanol und/oder Dimethylether in Kohlenwasserstoffe, für die Alkylierung, Disproportionierung und/oder Isomerisierung von Aromaten oder für die Aromatisierung von Olefinen verwendet.

Es kann auch vorteilhaft sein, insbesondere um hochaktive Katalysatoren für spezielle Zwecke zu erhalten, während oder nach dem Ionenaustausch weitere, die katalytischen Eigenschaften verbessernde Komponenten in das Aluminiumsilikat einzubringen. Als derartige Komponenten kommen beispielsweise Schwermetalle, Edelmetalle, Nichtmetallverbindungen und/oder Verbindungen der Seltenen Erden in Frage, die nach an sich bekannten Techniken eingebracht werden können.

Bei einer beispielhaften technischen Ausführungsform des erfindungsgemäßen Verfahrens kann wie folgt verfahren werden:

Kugelförmiges amorphes Aluminiumsilikat mit etwa 5 mm Partikeldurchmesser und einem Aluminiumoxidgehalt im Bereich 2 bis 15 Gew.-% wird bei einer Temperatur von über 800°C geglüht und danach im Vakuum entgast. Das so vorbehandelte Ausgangsmaterial wird mit einem Gemisch aus wäßriger Natronlauge und einer oder mehreren Stickstoffverbindungen versetzt, wobei die Menge dieses Gemisches einem Vielfachen des Porenvolumens des Ausgangsmaterials entspricht. Die Natronlauge kann beispielsweise 0,1 bis 10 Gew.-% Hydroxidionen (bezogen auf eingesetztes Aluminiumsilikat) enthalten. Als Stickstoffverbindungen können beispielsweise Tetrapropylammoniumhalogenide, Tetrabutylammoniumhalogenide, Hexamethylendiamin, Ethanolamin, Propylamin, Butylamin oder entsprechende quarternäre Ammoniumverbindungen in einer Menge von 5 bis 100 Gew.-% (bezogen auf eingesetztes Aluminiumsilikat) eingesetzt werden. In einem alkali- und druckbeständigen Reaktionsgefäß wird dann bei 100 bis 150°C im Verlaufe von 10 bis 25 Tagen das amorphe Aluminiumsilikat in weitgehend kristallines Aluminiumsilikat überführt. Nach Beendigung der Kristallisation wird die überschüssige

wäßrige Phase mechanisch abgetrennt. Durch Waschen mit Wasser entfernt man überschüssiges Alkali und trocknet das Reaktionsprodukt. Anschließend wird dieses bei 400 bis 600°C calciniert, dann so lange mit einer wäßrigen Ammoniumsalzlösung behandelt, bis der überwiegende Teil der im Reaktionsprodukt vorhandenen austauschbaren Natriumionen durch Ammoniumionen ersetzt ist. Dann wird nochmals getrocknet und calciniert und so ein Katalysator erhalten, der direkt in Festbettreaktoren eingefüllt werden kann, in denen beispielsweise Methanol und/oder Dimethylether in Kohlenwasserstoffe umgewandelt werden. Der Katalysator kann auch für die Aromatisierung von Olefinen, für das katalytische Cracken, für die Alkylierung und Disproportionierung von Aromaten oder für Adsorptionen eingesetzt werden. Der Katalysator ist, gegebenenfalls nach weiterer Modifizierung, auch für andere katalytische Reaktionen einsetzbar.

Die erfindungsgemäß hergestellten Katalysatoren sind wesentlich einfacher zugänglich als entsprechende Katalysatoren, die gemäß dem Stand der Technik aus löslichen oder teilweise löslichen Kieselsäuren oder Kieselsäurederivaten hergestellt werden. Die erfindungsgemäß hergestellten Katalysatoren haben eine solche mechanische Stabilität (pro Formkörper), daß sie auch bei technischem Dauereinsatz nicht zerfallen und somit der Druckverlust in Festbettreaktoren beim technischen Einsatz dieser Katalysatoren auch bei längeren Laufzeiten gering ist. Selbst thermische Regenerierungen, durch die im Verlauf der katalytischen Umwandlungen entstehende Koksbeläge oberhalb 500°C im Luftstrom abgebrannt werden können, beeinträchtigen die mechanischen Eigenschaften der Katalysatoren nur unwesentlich.

Weiterhin ist beim erfindungsgemäßen Verfahren vorteilhaft, daß keine Zusätze benötigt werden, um das gewünschte Verhältnis von Kieselsäure zu Aluminiumoxid einzustellen und daß die einzusetzende Wassermenge in weiten Grenzen variierbar ist.

Es ist ausgesprochen überraschend, daß die erfindungsgemäße Behandlung von Formkörpern aus amorphem Aluminiumsilikat nicht zum Zerfall der Formkörper bzw. zur Bildung von mechanisch wenig stabilen Formkörpern führt, denn bei der Behandlung mit basischen Mitteln konnte die Bildung von löslichen Silikaten bzw. Aluminaten erwartet werden und damit der Zerfall der eingesetzten Formkörper zumindest schon bei geringer mechanischer Belastung. Überraschenderweise besitzen die erfindungsgemäß hergestellten Katalysatoren eine gute mechanische Stabilität (Bruchhärte), trotz der Verwendung eines basischen und deshalb aggressiven Reaktionsmediums, das beispielsweise mehrere 100 Stunden bei erhöhter Temperatur einwirkt. Insbesondere war bei der Verwendung relativ stark basischer Verbindungen aus der Gruppe der Alkali- und Erdalkalihydroxide, der Alkalicarbonate, Alkaliphosphate, Alkalifluoride, Alkalisulfide, Alkalicarboxylate, Alkalisilikate und Alkaliwolframate zu erwarten, daß das Einsatzmaterial so angegriffen wird, daß es nicht zur Bildung formstabiler, für einen technischen Einsatz in Festbettreaktoren geeigneter Katalysatoren kommt.

Das erfindungsgemäße Verfahren und die Verwendung der dabei erhältlichen Produkte wird durch die folgenden Beispiele erläutert, ohne die Erfindung auf diese Beispiele einzuschränken.

## Beispiele

### Beispiel 1

Dieses Beispiel betrifft die Herstellung eines für die Umwandlung von Methanol und/oder Dimethylether in Kohlenwasserstoffe geeigneten Katalysators.

100 g eines kugelförmigen Katalysatorträgermaterials mit einem Durchmesser von ca. 5 mm auf Basis eines amorphen Aluminiumsilikats mit einem Gehalt von 1,65 Gew.-% Aluminiumoxid wurden durch 4-stündiges Glühen bei 900°C aktiviert und nach dem Abkühlen durch Evakuieren entgast.

Es wurde eine Lösung bestehend aus 4 g Natriumhydroxid, 140 ml Wasser und 26,6 g Tetrapropylammoniumbromid bereitet und damit das entgaste Ausgangsmaterial übergossen. Die Mischung wurde in ein Polypropylengefäß überführt und bei einer Temperatur von 100°C belassen. Nach 18 Tagen wies das ursprünglich amorphe Ausgangsmaterial eine gute Kristallinität auf, was durch eine Debye-Scherrer-Aufnahme festgestellt wurde.

Die Reaktionslösung wurde dann abdekantiert und das in kristallines Silikat umgewandelte Material so lange mit Wasser gewaschen, bis die ablaufende Waschlösung einen pH-Wert von 7 bis 8 aufwies. Das gewaschene Produkt wurde in Vakuum bei 120°C getrocknet und zur Entfernung organischer Anteile bei einer Temperatur von 550°C 4 Stunden lang calciniert.

Es resultierte die Natriumform des kristallinen Silikates, die ein molares Verhältnis von

$Na_2O:Al_2O_3:SiO_2$ wie 0,7:1,0:133,9

aufwies.

Durch Behandlung mit einem Überschuß an verdünnter Ammoniumchloridlösung wurde die Ammonium-Form erhalten, die bei einer Temperatur von 500°C thermisch zersetzt wurde.

Zur Überprüfung der katalytischen Aktivität wurden 30 ml des so hergestellten kugelförmigen Festbettkatalysators in einen elektrisch beheizten und mit einer Vorheizzone, sowie mit Einlaßvorrichtungen für Sticktoff und Methanol versehenen Quarzreaktor gefüllt und bei einer Temperatur von 370°C ohne Anwendung von Druck mit Methanol in Berührung gebracht. Bei einer Kontaktbelastung von 791 g Methanol pro Liter Katalysator und pro Stunde und nach einer Versuchdauer von 158 Stunden wurde folgendes Ergebnis erhalten (ohne Berücksichtigung des Reaktionswassers):

| | |
|---|---|
| Methanolumsatz: | 99,8 % |
| Produktzusammensetzung | (Gew.-%) |
| $H_2$: | 0,14 |
| $CH_4$: | 0,6 |
| $C_2H_6$: | 0,2 |
| $C_2H_4$: | 8,3 |
| $C_3H_8$: | 3,0 |
| $C_3H_6$: | 16,3 |

| | |
|---|---|
| $C_4H_{10}$: | 5,1 |
| $C_4H_8$: | 16,2 |
| $C_5^+$: | 32,0 |
| $CH_3$-O-$CH_3$: | 6,5 |
| Benzol: | 0,5 |
| Toluol: | 2,0 |
| $C_8$-Aromaten: | 6,6 |
| $C_9$-Aromaten: | 2,5 |

Beispiel 2

Dieses Beispiel zeigt die Eignung des nach Beispiel 1 hergestellten Materials als Katalysator für die Aromatisierung von Ethylen.

Das Katalysatormaterial wurde auf eine Korngröße von 1 bis 2 mm zerkleinert und in einen Mikroreaktor in einer Menge von 5 ml eingefüllt. Es wurde mit Stickstoff gespült und anschließend bei einer Temperatur von 300°C ein Strom von Ethylen in einer Menge von 540 ml pro Stunde über den Kontakt geleitet. Neben einem geringen Anteil einer nahezu ethylenfreien Gasphase wurde ein Flüssigprodukt mit folgender Zusammensetzung erhalten:

18,5 % $C_5^+$-Aliphaten
2,8 % Benzol
14,7 % Toluol
21,9 % $C_8$-Aromaten
42,1 % Hochsieder

0037982

Beispiel 3

Dieses Beispiel zeigt die Eignung des nach Beispiel 1 hergestellten Materials als Katalysator für die Disproportionierung von Aromaten.

Das Ausgangsmaterial Toluol wurde verdampft und bei einer Temperatur von 540°C kontinuierlich über den gemäß Beispiel 1 hergestellten Katalysator geleitet. Bei einer Kontaktbelastung von 1 Volumenteil Toluol pro Volumenteil Katalysator und pro Stunde und einem Druck von 0,5 bar wurde ein Reaktionsprodukt folgender Zusammensetzung erhalten:

8,0 % Benzol
80,6 % Toluol
11,4 % $C_8^+$-Aromaten

Beispiel 4

Dieses Beispiel betrifft die Herstellung eines Katalysators mit relativ hohem Aluminiumoxidgehalt.

Zum Einsatz gelangte ein handelsübliches silikatisches Material in der Form von Kugeln mit 5 mm Durchmesser, das eine Zusammensetzung von

$SiO_2$: 69,8 %
$Al_2O_3$: 14,98 %
$Fe_2O_3$: 2,97 %

$Na_2O$: 0,24 %
(Differenz zu 100 %: $H_2O$)

aufwies.

5 kg dieses Materials wurden 6 Stunden bei 900°C geglüht. Nach Abkühlung und Entgasung durch Evakuieren wurde es mit einer Lösung, bestehend aus

1,0 kg Tetrapropylammoniumbromid
0,4 kg NaOH und
7,0 kg Wasser

getränkt.

Die Mischung wurde in einen Stahlautoklaven überführt und ohne Rühren 15 Tage bei einer Temperatur von 130°C gehalten. Nach dem Auswaschen, Trocknen und Calcinieren wurde ein partiell kristallines Material erhalten, das folgende molare Zusammensetzung aufwies:

$$(Na_2O)_{0,24} \cdot (Al_2O_3)_{1,0} \cdot (SiO_2)_{7,6}$$

Beispiel 5

Dieses Beispiel zeigt die Eignung des nach Beispiel 4 hergestellten Katalysators für die Umwandlung von Methanol in niedere Olefine.

30 ml des gemäß Beispiel 4 hergestellten und gemäß Beispiel 1 Ammonium-ausgetauschten und calcinierten Kontaktes wurden bei einer Temperatur von 370°C ohne Anwendung von

Druck in einem Quarzreaktor mit gasförmigem Methanol beschickt. Die Methanolzufuhr wurde so gewählt, daß eine Kontaktbelastung von einem Volumenteil Methanol pro Volumenteil Katalysator und pro Stunde resultierte.

Nach einer Versuchsdauer von 24 Stunden betrug bei einem Methanolumsatz von 62 % ohne Berücksichtigung des Reaktionswassers die Produktzusammensetzung (Gew.-%):

| | |
|---|---|
| $H_2$: | 0,01 |
| $CH_4$: | 0,4 |
| $C_2H_6$: | 0,01 |
| $C_2H_4$: | 4,3 |
| $C_3H_8$: | 0,2 |
| $C_3H_6$: | 4,9 |
| $C_4H_{10}$: | 0,6 |
| $C_4H_8$: | 2,6 |
| $C_5^+$: | 4,2 |
| $CH_3-O-CH_3$: | 81,3 |
| Aromaten: | 1,4 |

Da der im Reaktionsgemisch enthaltene Dimethylether rezirkulierbar ist und anstelle von Methanol als Einsatzprodukt dienen kann, errechnet sich eine Kohlenstoff-Selektivität zu $C_2$- bis $C_4$-Olefinen von ca. 66 %.

Beispiel 6

Dieses Beispiel verdeutlicht, daß beim erfindungsgemäßen Verfahren eine breite Variationsmöglichkeit hinsichtlich der einsetzbaren organischen Stickstoffverbindungen gegeben ist.

Es wurden 100 g eines handelsüblichen, amorph-silikatischen und geformten Materials, das gemäß Spezifikation einen Aluminiumoxidgehalt von unter 5 % aufwies, mit einer Lösung von 0,1 Mol Tris-(2-hydroxyethyl)-n-propylammoniumhydroxid und 0,1 Mol Natriumhydroxid zu 125 ml in Wasser getränkt und die Mischung für eine Dauer von 3 Wochen bei einer Temperatur von 100°C belassen.

Nach Aufarbeitung entsprechend Beispiel 1 wurde ein kristallines Aluminiumsilikat enthaltendes Material erhalten, das folgende molare Zusammensetzung aufwies:

$(Na_2O)_{0,24}\ (Al_2O_3)_{1,0}\ (SiO_2)_{40,97}$

Beispiel 7

Dieses Beispiel zeigt, daß sich der nach Beispiel 6 hergestellte und gemäß Beispiel 1 Ammonium-ausgetauschte und calcinierte Katalysator zur Herstellung von Olefinen aus Methanol eignet.

Bei 370°C und einer Kontaktbelastung von 790 g Methanol pro Liter Katalysator und pro Stunde wurde Methanol ohne Anwendung von Druck zu 85 % in Wasser und ein Reaktionsprodukt umgewandelt, das folgende Zusammensetzung aufwies:

$H_2$: 0,1 %
$CH_4$: 0,5 %
$C_2H_4$: 6,1 %
$C_3H_8$: 0,3 %
$C_3H_6$: 13,9 %
$C_4H_{10}$: 1,2 %
$C_4H_8$: 9,3 %
$C_5^+$: 25,6 %
$CH_3-O-CH_3$: 37,6 %
Aromaten: 5,4 %

Im Reaktionsprodukt konnte kein Kohlenmonoxid und kein Ethan nachgewiesen werden.

Da Dimethylether zurückgeführt und als Einsatzprodukt anstelle von oder in Mischung mit Methanol verwendet werden kann, errechnet sich eine Kohlenstoff-Selektivität zu leichten ($C_2$- bis $C_4$-)Olefinen von ca. 51 %.

Beispiel 8

Dieses Beispiel veranschaulicht die Umwandlung eines handelsüblichen, amorphen Aluminiumsilikats in einen für die Herstellung von Kohlenwasserstoffen aus Dimethylether geeigneten Katalysator .

2000 g eines kugelförmigen Katalysator-Trägermaterials vom Typ KA-1, das einen Durchmesser von 5 mm und laut Spezifikation einen Gehalt an Aluminiumoxid von weniger als 5 % aufwies, wurden durch 8-stündiges Glühen bei 800°C aktiviert und nach dem Abkühlen bei Raumtemperatur durch Evakuieren entgast. Das so vorbehandelte Einsatzmaterial wurde mit einer Lösung, bestehend aus

60 g Natriumcarbonat
100 g Natriumhydroxid
2800 ml Wasser
524 g Tetrapropylammoniumbromid

getränkt und für die Dauer von 3 Wochen einer Temperatur von 95°C ausgesetzt. Die Aufarbeitung des umgewandelten Produktes geschah wie in Beispiel 1 beschrieben.

Das umgewandelte Katalysatormaterial wies Röntgenbeugungslinien auf, die es als kristallines Natrium-Aluminium-Silikat charakterisierten. Die Zusammensetzung betrug laut Analyse:

93,9 % $SiO_2$;
1,74 % $Al_2O_3$;
1,52 % $Na_2O$.
(Differenz zu 100 %: = $H_2O$).

Nach weitgehendem Austausch der Natriumionen gegen Ammoniumionen und anschließender Dekationisierung nach bekannten Techniken wurde der so aktivierte Katalysator für die Umwandlung von Dimethylether im fest angeordneten Katalysatorbett verwendet. Zum Einsatz gelangten 120 ml des kugelförmigen, kristallinen Aluminiumsilikats unter den Versuchsbedingungen:

Temperatur: 370°C
Druck: 1,23 bar
Dimethylethereinsatz: 579,86 g pro 1 Katalysator und pro Stunde.

So konnte bei einem Umsatz von 98 % ein Produktgemisch der folgenden Zusammensetzung erhalten werden (Angaben

in Gew.-% und ohne Berücksichtigung des Reaktionswassers):

Benzol: 1,3

Toluol: 4,2

$C_8$-Aromaten: 15,3

$C_9^+$-Aromaten: 5,7

$H_2$: 0,1

$CH_4$: 0,6

$C_2H_6$: 0,2

$C_2H_4$: 2,1

$C_3H_8$: 5,3

$C_3H_6$: 3,5

n-$C_4H_{10}$: 2,2

i-$C_4H_{10}$: 7,9

n-$C_4H_8$: 2,8

i-$C_4H_8$: 2,8

$C_5^+$: 43,0

$CH_3OCH_3$: 3,0

Beispiel 9

Dieses Beispiel zeigt, daß für die Umwandlung der erfindungsgemäß verwendeten Formkörper in kristallines Aluminiumsilikat mit hoher Bruchhärte ein wäßriges Milieu geeignet ist, dessen Basizität vorwiegend durch hydrolysierende Salze aufrechterhalten wird.

Ausgangsmaterial war das in Beispiel 1 verwendete amorphe und geformte Aluminiumsilikat, das wie dort beschrieben vorbehandelt und getränkt wurde. Kristallisationsdauer und -temperatur betrugen in allen Fällen 30 Tage und 110°C. Die Zusammensetzung der wäßrigen, hydrolysierbaren Salze enthaltenden Lösung und die Analyse des jeweils erhaltenen kristallinen Materials ist aus Tabelle 1 ersichtlich.

Tabelle 1

| Versuch Nr. | I | II | III | IV | V |
|---|---|---|---|---|---|
| Eingesetzte Menge amorphes Aluminiumsilikat | 5 kg | 100 g | 100 g | 100 g | 100 g |
| Zusammensetzung der wäßrigen Lösung: | | | | | |
| hydrolysierbares Salz | Natronwasserglas *) 3,6 kg | $Na_2WO_4$ 29,3 g | $Na_2S.9H_2O$ 7,8 g | $Na_2CO_3$ 15,9 g | $Na_3PO_4 \cdot 12H_2O$ 38,01 g |
| Tetrapropylammoniumbromid | 665 g | 26,6 g | 26,6 g | 26,6 g | 26,6 g |
| Wasser | 4,0 kg | 140 ml | 140 ml | 140 ml | 140 ml |
| Sonstiges | - | 2 g NaOH | - | - | - |
| Analyse des erhaltenen kristallinen Materials**) | | | | | |
| $SiO_2$ | 97,2 % | 92,3 % | 91,8 % | 91,4 % | 92,6 % |
| $Al_2O_3$ | 2,04 % | 2,87 % | 3,32 % | 3,17 % | 2,74 % |
| $Na_2O$ | 0,28 % | 0,94 % | 0,36 % | 0,89 % | 0,93 % |
| Struktur | kristallin | kristallin | kristallin mit amorphem Anteil | kristallin | kristallin |

*) 8,6 % $Na_2O$, 25,4 % $SiO_2$

**) Differenz zu 100 % = $H_2O$

Die nach Beispiel 9 hergestellten kristallinen Silikate zeichneten sich durch eine hohe mechanische Festigkeit aus, die weitgehend der des Ausgangsmaterials entsprach und die für einen technischen Einsatz in jeder Hinsicht ausreichend war.

Die katalytischen Eigenschaften dieser kristallinen Silikate sind aus Beispiel 10 ersichtlich.

Beispiel 10

Die katalytische Aktivität der nach Beispiel 9 hergestellten und gemäß Beispiel 1 Ammonium-ausgetauschten und calcinierten Katalysatoren wurde für die Umwandlung von Methanol in Kohlenwasserstoffe überprüft. Dabei wurden bei einer Temperatur von 370°C, einem Druck von 1 bar, bei einer Kontaktbelastung von 791 g Methanol pro l Katalysator und pro Stunde nach einer Versuchsdauer von 24 Stunden folgende Ergebnisse erhalten:

| Katalysator | I | II | III | IV | V |
|---|---|---|---|---|---|
| Produktzusammensetzung in Gew.-% | | | | | |
| (ohne Berücksichtigung des Reaktionswassers) | | | | | |
| $C_6^+$ Aromaten | 14,2 | 26,6 | 4,9 | 27,5 | 23,3 |
| $H_2$, CO, $CO_2$, $CH_4$ | 1,4 | 1,6 | 0,5 | 0,8 | 2,2 |
| $C_2H_6$ | 0,3 | 0,5 | - | 0,3 | 0,9 |
| $C_2H_4$ | 10,6 | 3,9 | 8,7 | 4,2 | 7,0 |
| $C_3H_8$ | 4,1 | 9,0 | 0,4 | 7,9 | 9,8 |
| $C_3H_6$ | 18,3 | 8,0 | 12,6 | 2,5 | 11,2 |
| $C_4$ | 14,3 | 20,4 | 4,9 | 21,5 | 20,6 |
| $C_5^+$ | 14,1 | 23,9 | 19,7 | 30,9 | 17,1 |
| $CH_3$-O-$CH_3$ | 22,7 | 6,1 | 48,3 | 4,4 | 7,9 |
| Methanolumsatz (%) | 90,1 | 99,1 | 83,2 | 99,9 | 99,8 |

Beispiel 11 (Vergleichsbeispiel)

Dieses Vergleichsbeispiel verdeutlicht die technischen Probleme, die auftreten können, wenn ein nach konventioneller Synthese hergestelltes, kristallines Aluminiumsilikat als Festbettkatalysator verwendet werden soll.

Unter Verwendung der Ausgangsmaterialien Aluminiumoxid, Kieselsol, Natronlauge, Wasser und Monoethanolamin wurde gemäß der europäischen Patentanmeldung mit der Veröffentlichungs-Nr. 0002899, Beispiel 1, ein kristallines Aluminiumsilikat hergestellt.

Bei dem Reaktionsprodukt handelte es sich um staubförmiges Material, das aufgrund seiner mikrokristallinen Beschaffenheit nicht als Festbettkatalysator verwendet werden konnte.

Dieses Material wurde mit einem Blauton als Bindemittel im Verhältnis 5:1 versetzt und mit Wasser zu einer extrudierbaren Paste verarbeitet. Es resultierte nach Verformung, Trocknung, Calcinierung, Ionenaustausch ($Na^+$ gegen $H^+$) und nochmaliger Trocknung und Calcinierung ein geformter Katalysator von bröckeliger Beschaffenheit, d.h. relativ hoher Empfindlichkeit gegen mechanische Beanspruchung.

Mit diesem Katalysator konnten bei einer Temperatur von 370°C, einem Druck von 1 bar und einer Methanolzufuhr von 791 g pro l Katalysator und pro Stunde folgende Versuchsergebnisse erhalten werden:

0037982

| Versuchsdauer (Stunden) | 4 | 8 | 12 | 24 | 36 |
|---|---|---|---|---|---|
| Methanolumsatz (Gew.-%) | 99,9 | 99,7 | 97,7 | 64,9 | 36,4 |
| Produktzusammensetzung in Gew.-% | | | | | |
| (ohne Berücksichtigung des Reaktionswassers) | | | | | |
| $C_6^+$-Aromaten | 35,9 | 34,8 | 31,0 | 18,9 | 0,0 |
| $CO$, $H_2$, $CH_4$, $CO_2$ | 8,3 | 3,0 | 2,5 | 1,6 | 2,4 |
| $C_2H_6$ | 1,3 | 0,5 | 0,5 | 0,1 | 0,1 |
| $C_2H_4$ | 1,3 | 2,6 | 5,7 | 0,1 | 0,2 |
| $C_3H_8$ | 18,0 | 12,5 | 9,9 | 0,0 | 0,0 |
| $C_3H_6$ | 1,9 | 2,8 | 5,3 | 0,1 | 0,1 |
| $C_4H_{10}$ | 19,6 | 20,0 | 17,3 | 0,0 | 0,0 |
| $C_4H_8$ | 5,0 | 6,5 | 7,0 | 0,2 | 0,0 |
| $C_5^+$ | 6,6 | 15,5 | 14,4 | 5,1 | 0,0 |
| $CH_3$-O-$CH_3$ | 2,1 | 1,9 | 6,4 | 73,9 | 97,2 |

Dieses Versuchsergebnis zeigt, daß dieser auf konventionelle Weise hergestellte Festbettkatalysator zwar eine hohe Anfangsaktivität besitzt, aber schon nach einer Versuchszeit von weniger als 24 Stunden überwiegend Dimethylether produziert. Nach einer Versuchsdauer von 36 Stunden sind außer $C_1$-Verbindungen und Spuren leichter Olefine keine der erwünschten Kohlenwasserstoffe (Olefine, Aromaten, $C_5^+$) im Reaktionsprodukt mehr enthalten.

Demgegenüber zeigen die erfindungsgemäß hergestellten Katalysatoren neben der mechanischen Beständigkeit eine wesentlich länger andauernde Aktivität.

Beispiel 12

Dieses Beispiel betrifft die Verwendung eines Amin-Phosphat-Gemisches bei der Herstellung eines geformten Festbettkatalysators aus amorphem, vorgeformtem Aluminiumsilikat.

100 g eines handelsüblichen amorph-silikatischen Katalysator-Trägermaterials vom Typ KA-2, das in Form von Kugeln mit 5 mm Durchmesser vorliegt und das nach Spezifikation einen Aluminiumoxidgehalt von weniger als 5 % aufwies, wurden während der Dauer von 4 Stunden bei 900°C calciniert und nach Abkühlung auf Raumtemperatur durch Evakuieren entgast.

Es wurde eine Lösung bestehend aus 20 g Hexamethylendiamin, 16,4 g Trinatriumphosphat und 140 g Wasser bereitet, mit der das entgaste Ausgangsmaterial übergossen wurde.

Das gesamte Reaktionsgemisch wurde in einen Stahlautoklaven überführt und ohne Rühren einer Temperatur von 180°C ausgesetzt. Nach einer Dauer von 140 Stunden wurde abgekühlt, und, wie in Beispiel 1 beschrieben, aufgearbeitet.

Mittels einer Debye-Scherrer-Aufnahme konnte im Reaktionsprodukt ein hoher Anteil von Zeolith ZSM-5 nachgewiesen werden.

Im Vergleich mit dem Ausgangsmaterial wies das kristallisierte Reaktionsprodukt nahezu identische geometrische Abmessungen auf. Es besaß weiterhin eine hohe und für

einen technischen Einsatz voll ausreichende Bruchhärte.

Beispiel 13

Die Aktivität des nach Beispiel 12 hergestellten und nach Beispiel 1 aktivierten Katalysators wurde hinsichtlich der Umwandlung von Methanol in Kohlenwasserstoffe unter den gleichen Versuchsbedingungen, wie in Beispiel 1 angegeben, überprüft. Es wurden folgende Versuchsergebnisse erhalten:

| Versuchsdauer (Stunden) | 1 | 8 | 24 | 48 | 60 |
|---|---|---|---|---|---|
| Methanolumsatz (Gew.-%) | 100 | 100 | 99 | 95 | 97 |
| Produktzusammensetzung *) in Gew.-% (ohne Berücksichtigung des Reaktionswassers) | | | | | |
| $C_5^+$-Kohlenwasserstoffe | 18,6 | 18,9 | 28,7 | 37,0 | 45,6 |
| Benzol | 2,3 | 2,2 | 1,7 | 1,8 | 2,0 |
| Toluol | 13,6 | 13,9 | 11,1 | 5,9 | 3,8 |
| $C_8$-Aromaten | 38,4 | 41,4 | 36,0 | 28,7 | 22,8 |
| $C_9^+$-Aromaten | 27,1 | 23,6 | 22,5 | 26,6 | 25,8 |

*) des bei Raumtemperatur und Normaldruck flüssigen Anteils organischer Produkte

Der Versuch wurde nach einer Gesamtversuchsdauer von 100 Stunden unterbrochen. Zu dieser Zeit besaß der Katalysator noch eine gute Aktivität. Es wurde festgestellt, daß die einzelnen Formkörper nach der Gesamtversuchsdauer noch eine unverändert hohe Bruchhärte aufwiesen.

Patentansprüche

1. Verfahren zur Herstellung von geformten Katalysatoren mit einem minimalen Durchmesser über 1 mm und einem maximalen Durchmesser von unter 20 mm auf der Basis kristalliner Aluminiumsilikate, dadurch gekennzeichnet, daß man Formkörper mit einem minimalen Durchmesser von über 1 mm und einem maximalen Durchmesser von unter 20 mm, die im wesentlichen aus amorphem Aluminiumsilikat mit einem Aluminiumoxidgehalt von 0,1 bis 20 Gew.-% bestehen, zusammen mit aliphatischen $C_2$- bis $C_{24}$-Aminen oder cycloaliphatischen $C_4$- bis $C_{24}$-Aminen und/oder Ammoniumsalzen entsprechend diesen aliphatischen und/oder cycloaliphatischen Aminen und/oder den aus diesen Aminen durch Quarternierung erhältlichen quarternären Ammoniumverbindungen, in einem wäßrigen Milieu, bei einem pH-Wert von größer als 8, Temperaturen von 50 bis 250°C aussetzt und anschließend auswäscht, trocknet und bei 400 bis 600°C calciniert.

2. Verfahren nach Anspruch 1, dadurch gekennzeichnet, daß man Formkörper, die im wesentlichen aus amorphem Aluminiumsilikat mit einem Aluminiumgehalt von 1 bis 15 Gew.-% bestehen, einsetzt.

3. Verfahren nach Ansprüchen 1 und 2, dadurch gekennzeichnet, daß dem wäßrigen Milieu Amine, anorganische Oxide, anorganische Hydroxide und/oder Salze aus starken Basen und schwachen Säuren zugesetzt werden.

0037982

4. Verfahren nach Ansprüchen 1 bis 3, dadurch gekennzeichnet, daß dem wäßrigen Milieu Alkalicarbonate, Alkaliphosphate, Alkalifluoride, Alkalisulfide, Alkalicarboxylate, Alkalisilikate und/oder Alkaliwolframate zugesetzt werden.

5. Verwendung von gemäß den Ansprüchen 1 bis 4 hergestellten Katalysatoren als Festbettkatalysatoren für die Umwandlung von Methanol und/oder Dimethylether in Kohlenwasserstoffe, als Crackkontakte, für die Alkylierung, Disproportionierung und/oder Isomerisierung von Aromaten oder für Aromatisierung von Olefinen.

0037982

Europäisches Patentamt

# EUROPÄISCHER RECHERCHENBERICHT

Nummer der Anmeldung

EP 81 10 2514

| EINSCHLÄGIGE DOKUMENTE | | | KLASSIFIKATION DER ANMELDUNG (Int. Cl.) |
|---|---|---|---|
| Kategorie | Kennzeichnung des Dokuments mit Angabe, soweit erforderlich, der maßgeblichen Teile | betrifft Anspruch | B 01 J 29/28<br>C 01 B 33/28 |
| | DE - A - 2 643 928 (IMPERIAL CHEMICAL INDUSTRIES)<br>* Ansprüche 5 und 7; Seite 11, Zeilen 7-24; Seite 12, Zeilen 16-30; Beispiele 1 und 5 *<br>--- | 1,3,5 | |
| | US - A - 3 422 033 (H.P. BALLARD et al.)<br>* Insgesamt *<br>--- | 1-3 | |
| A | US - A - 3 515 682 (W.H. FLANK et al.)<br>* Spalte 6, Zeilen 27-41 *<br>--- | 1 | RECHERCHIERTE SACHGEBIETE (Int. Cl.) |
| A | GB - A - 1 099 073 (UNIVERSAL OIL PRODUCTS Co.)<br>& DE - A - 1 567 861 | | B 01 J 29/28<br>C 01 B 33/28<br>B 01 J 29/06 |
| A | US - A - 4 091 007 (F.G. DWYER et al.) | | |
| A | DE - B - 1 467 129 (MOBIL OIL CORP.)<br>--------- | | |

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 20.07.1981 | CECCHINI |

EPA form 1503.1 06.78